# EUROPEAN PATENT SPECIFICATION

(11) **EP 0 947 208 B1**
(45) Date of publication and mention of the grant of the patent: **14.07.2004**
(21) Application number: 99302424.9
(22) Date of filing: 29.03.1999
(51) Int. Cl.: A61M 5/168, A61M 5/14

(54) **Medical fluid supplying apparatus**
Abgabevorrichtung für medizinische Flüssigkeiten
Dispositif de distribution de liquide médical

(30) Priority: 01.04.1998 JP 8867798
(43) Date of publication of application: 06.10.1999
(73) Proprietor: AUBEX CORPORATION, Sumida-ku, Tokyo 130 (JP)
(72) Inventor: Sakuda, Ryutaro, Tokyo (JP); Kanai, Masahiro, Tokyo (JP)
(74) Representative: Haley, Stephen

(56) References cited:
- EP-A- 0 012 445
- EP-A- 0 800 837
- DE-A- 4 430 422
- US-A- 5 207 643
- US-A- 5 318 515
- US-A- 5 360 411

## Description

The present invention relates to a fluid supplying apparatus which continuously and gradually supplies fluid contained within a fluid container. More specifically, it relates to a fluid supplying apparatus for continuously supplying medical fluid, transfusion and so on to a living body such as a human body and an animal, for continuously supplying plants with water, nutrients (fluid), medical fluid (an insecticide fluid) and so on, and for continuously supplying a fish aquarium with medical fluid such as an antibiotic, bait (fluid), nutrients (fluid) for water plants and so on.

A medical fluid injection apparatus, for instance, uses a tube with small diameter having conduit function and flow rate control function and the medical fluid is supplied to the tube so that it is continuously injected into human body little by little.

Conventionally, flow rate of the medical fluid injection apparatus having the conduit function and the flow rate control function is changed by exchanging the tube, which makes it impossible to change the flow rate immediately.

Accordingly, for overcoming above disadvantage, an arrangement where a flow rate switching device is connected to the outflow hole of medical fluid container through two tubes having different fluid passage rate (Japanese Patent Application Laid-Open No. Hei 6-121835) and another arrangement where a flow rate switching device is inserted at a halfway of a tube connecting the medical fluid container and a human body (Japanese Patent Publication of Examined Application No. Hei-9-225028) are proposed.

In the former flow rate changing arrangement, the outflow hole of medical fluid pump is branched in two-way, where one end of the two tubes is connected to each branch hole and a flow rate switching cock for selectively opening and closing either one of the two tubes is connected to the other end of the two tubes.

In the latter flow rate changing arrangement, an inflow portion and an outflow portion of the medical fluid is formed to a body case. The inflow portion and the outflow portion is connected through a plurality of tubes branched from a flow path branch in the body case and an operation valve is provided to the flow path branch for selectively opening and closing respective valves. A storing space is formed around the flow path branch in the body case, in which the plurality of tubes is stored in revolving manner around the flow path branch in the storing space.

However, there are the following disadvantages in the above-described conventional arrangement.

In the former arrangement, since the two tubes are composed of independent body, it is difficult to be handled in carrying and the tubes can be an encumbrance. Especially, since respective tubes have a certain length for securing the flow rate control function, it is inconvenient for handling and can be an encumbrance for storing.

In the latter arrangement, since the flow rate switching device is provided at the halfway of the tube, the tube becomes partially thick, thereby impairing usability.

US-A-5207643 discloses a multi-lumen catheter flow valve system.

The object of the present invention is to overcome the aforesaid disadvantage and to provide a fluid supplying apparatus convenient for carrying and storing and superior in usability, and which can easily change the flow rate in multi-stage.

According to the present invention there is provided a fluid supplying apparatus as defined by claim 1.

Since the fluid contained in the fluid container can be selectively circulated to the plurality of flow paths, the flow rate can be changed by selecting the flow path. For instance, when the fluid passage rate of the respective flow paths is the same, the flow rate can be changed by selecting the number of the selected flow paths. Accordingly, the flow rate can be easily changed in a multi-stage. Further, since the plurality of flow paths are provided in a single fluid supplying tube, the fluid supplying apparatus is not inconvenient for carrying and storing the usability thereof is not deteriorated.

Though the plurality of flow paths may have the same fluid passage rate, each of the plurality of flow paths may differ in the fluid passage rate so that the flow rate can be changed by selecting one flow path.

According to the above arrangement, the flow path can be selected for use by selectively shutting the plurality of flow paths with the closing area by rotating the flow path opening plate, thereby conducting the flow rate change with a simple operation.

In the above, the plurality of flow paths is preferably formed of a thermoplastic-resin-made tube element of a predetermined length having an opening with deformed cross-section.

In the accompanying drawings:
Fig. 1 is a perspective view showing a preferred embodiment of a fluid supplying apparatus according to the present invention;
Fig. 2 is a cross-section of the aforesaid embodiment;
Fig. 3 is a cross-section of a protection case of the aforesaid embodiment;
Fig. 4 is a cross-section of a water-repellant breathable filter of the aforesaid embodiment;
Fig. 5 is a cross-sectional view showing a cross-section of a tube of the apparatus of the aforesaid embodiment;
Fig. 6(A) to 6(E) are cross-sectional views showing different configurations of cross-sections of tube elements of the apparatus of the aforesaid embodiment;
Fig. 7 is a cross-section of flow rate switching device of the apparatus of the aforesaid embodiment;
Fig. 8 is an exploded perspective view showing flow rate switching device of the apparatus of the aforesaid embodiment;
Fig. 9 is an illustration of injecting medical fluid to the apparatus of the aforesaid embodiment;
Fig. 10 is a cross-section showing relationship between a protection case and rubber elastic film when the medical fluid is injected into the apparatus of the aforesaid embodiment;
Fig. 11(A) and 11(B) are illustrations for showing flow rate condition change in the flow rate switching device of the apparatus of the aforesaid embodiment; and
Fig. 12(A) and 12(B) are cross-sections of other examples of tubes for the apparatus of the aforesaid embodiment.

A preferred embodiment of the present invention will be described below with reference to drawings.

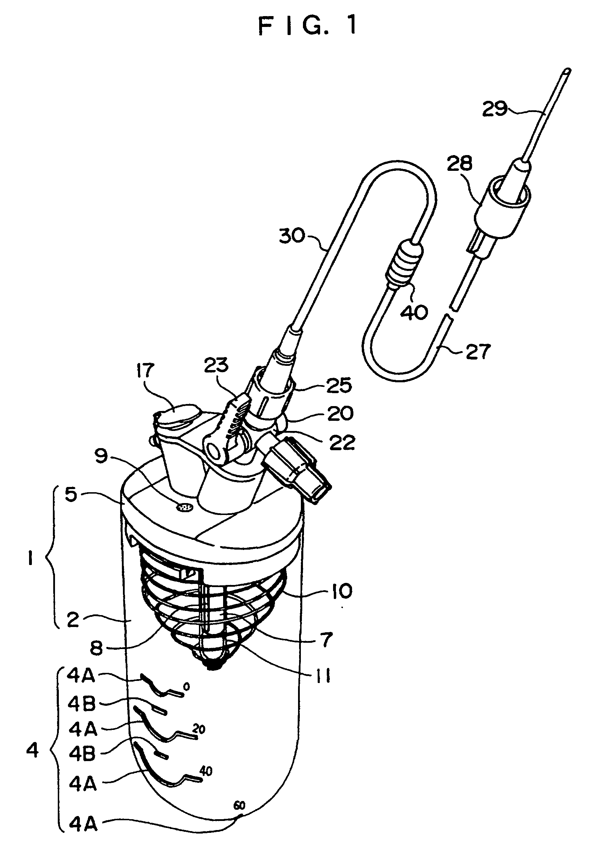

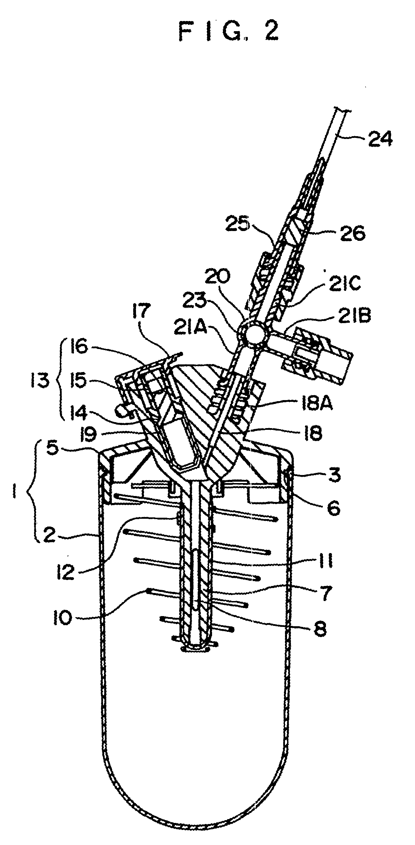

In the present embodiment, the present invention is applied to a medical fluid injection apparatus for injecting medical fluid to human body. Fig. 1 is a perspective view thereof, and Fig. 2 is a cross-sectional view thereof.

In the figures, a protection case 1 is composed of a bottomed cylindrical body 2 of transparent material such as plastic and glass, a lid body 5 made of polypropylene and fitted to an open end of the cylindrical body 2.

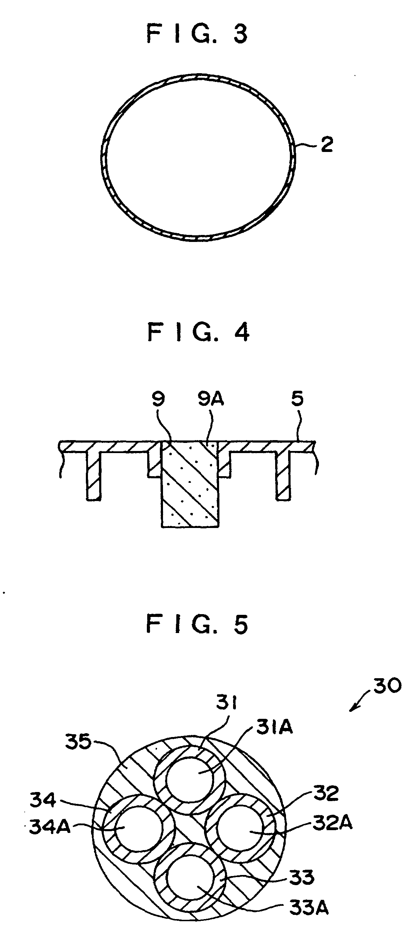

The cylindrical body 2 is formed in a bottomed cylinder having an inner configuration of deformed cross-section except for circle, an oval here (see Fig. 3), and is provided with a projection 3 on inner side adjacent to the open end and a scale 4 on outer side, respectively. The scale 4 indicates medical fluid containing amount (medical fluid containing amount inside below-mentioned rubber elastic film 11) by cc unit from intermediate position in the up and down direction toward bottom. The scale 4 is composed of even number scale 4A of "0", "20", "40" and "60" and odd number scale 4B of "10", "30" and "50".

The lid body 5 has an engaging concave portion 6 for engaging the projection 3 of the cylindrical body 2 at an outer side thereof, an air vent 9 and a medical fluid introduction tube 7 as a thin fluid introduction tube extending toward inside of the cylindrical body 2 substantially at the center of an upper side thereof. Both ends of the medical fluid introduction tube 7 are opened and a plurality of slits 8 is provided on circumference thereof as shown in Fig. 4. The air vent 9 has a water-repellant breathable filter 9A for circulating air inside and outside of the protection case 1 and preventing the medical fluid from permeating. A chemical-resistant synthetic resin bundle with water-repellant processing is preferably used as the water-repellant breathable filter 9A, for instance.

A rubber elastic film 11 with bottomed-tube shape is fitted in close contact with the medical fluid introduction tube 7, an open end of the rubber elastic film 11 being held by a pinch 12. An outer diameter and length of the medical fluid introduction tube 7 is substantially the same as an inner diameter and length of the contracted rubber elastic film 11. A maximum of 60cc medical fluid can be contained in the rubber elastic film 11. Incidentally, ordinarily approximate 20cc medical fluid is injected for cancer pain treating per one day, so that medical fluid for approximately three days can be contained therein.

The rubber elastic film 11 is expanded in accordance with injecting and receiving the medical fluid. A spring 10 stretching in proportion to the expansion of the rubber elastic film 11 is disposed at outer side thereof. The spring 10 is made of wire material having diameter of 0.6mm to 0.8mm, for instance. The spring 10 has an upper end stopped to the lid body 5 and is wound in a spiral manner so that the diameter thereof is gradually narrowed downward. The lowermost end is abutted to a pointed end of the medical fluid introduction tube 7 through the rubber elastic film 11.

The rubber elastic film 11 is preferably made of a chemical-resistant material undamaged by a function of medical fluid and having great toughness and stretchability, and transparent or translucent material is especially preferable. For example, silicone rubber and latex rubber on the market is preferable. The thickness of the rubber elastic film is approximately 0.4mm. A contraction power when the medical fluid is introduced in the rubber elastic film 11 is preferably 1000 to 7000mmAq (millimeter by water head) pressure. Since venous pressure of human body is ordinarily around 60mmAq, the medical fluid can be introduced to a patient by a pressure more than 60mmAq. When the contraction power of the rubber elastic film 11 falls below 1000mmAq, it is difficult to be controlled. When the contraction power exceeds 7000mmAq, the medical fluid is difficult to be injected from the syringe into the rubber elastic film 11 by human power. However, the contraction power is not limited to the range described above.

An inflow hole 19 as a fluid inflow hole for injecting the medical fluid into the rubber elastic film 11 and an outflow hole 18 as fluid outflow hole for discharging the medical fluid received inside the rubber elastic film 11 are provided adjacently in V-shape on an upper portion of the medical fluid introduction tube 7 (lid body 5). In other words, the inflow hole 19, the outflow hole 18 and the medical fluid introduction tube 7 is provided to the lid body 5 in substantially Y-shaped arrangement and mutually in communication.

A check valve 13 for allowing the inflow to the medical fluid introduction tube 7 from the outside and preventing the outflow from the medical fluid introduction tube 7 toward outside is provided inside the inflow hole 19. The check valve13 has a valve cylinder 15 buried in the inflow hole 19 and having a valve seat 14 at the halfway thereof, and a chemical-resistant valve bar 16 made of silicone rubber and the like and retractably accommodated in the valve cylinder 15 to open and close the valve seat 14. Incidentally, a cap 17 can be detachably attached to an outer end of the valve cylinder 15. A spiral groove 18A for detachably engaging a three-direction valve 20 is formed around the outflow hole 18. The three-direction valve 20 has a valve body 22 with three switch holes 21A, 21B and 21C, and a cock 23 for switching the flow path.

A connector 25 provided on one end of a fluid delivering tube 30 having conduit function and flow rate control function is detachably connected to the switching hole 21C of the three direction valve 20. A filter 26 for removing dust etc. in the medical fluid is accommodated inside the connector 25. A flow rate switching device 40 as a flow path selecting means is connected to the other end of the tube 30 and a connector 28 similar to the connector 25 is connected to the flow rate switching device 40 through a tube 27 having therein a single flow path. A syringe needle 29 is detachably attached to a distal end of the connector 28 as an attachment to human body. Accordingly, the inside of the rubber elastic film 11 and the syringe needle 29 are connected through the fluid delivering tube 30, flow rate switching device 40 and the tube 27.

The tube used for the fluid delivering tube 30 is formed in a predetermined length and has thereinside a plurality of flow paths extending parallel along a longitudinal direction thereof. Specifically, as shown in Fig. 5, the tube includes a plurality of (four, in the present embodiment) thermoplastic-resin made tube elements 31, 32, 33 and 34 of a predetermined length respectively having flow paths 31A, 32A, 33A and 34A of different fluid passage rate. The tube elements 31, 32, 33 and 34 are bundled and outer surface thereof is unitedly covered with a covering member 35.

Respective tube elements 31 to 34 may be a single-layered tube, or alternatively, a covered tube considering reinforcement and handling. All of Polypropylene (PP), polyethylene (PE), polyacetals (POM), polycarbonate (PC), ABS, polyamide resin, and polystylene (PS) can be used for a material of the tube element 31 to 34, however, transparent material is preferable. A flexible material is preferable for the covering member as a cover such as thermoplastic resin elastomer, polyolefin (LDPE, LLDEP) type elastomer, thermoplastic polyurethane elastomer, soft vinyl chloride resin and EVA.

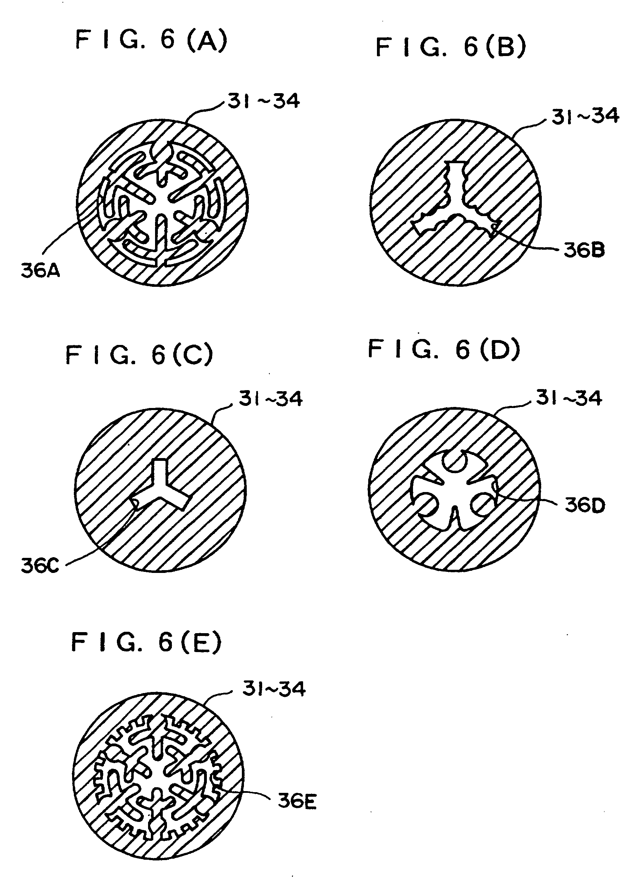

The configuration of the cross-section of the tube element 31 to 34 is deformed unlike a circular opening of conventional flow rate control means. Some examples are shown in Fig. 6.

An opening 36A of the tube elements 31 to 34 shown in Fig. 6(A) has three branch-shaped projections of different two types alternatively projecting from an inner circumference of a circular base hole toward the center thereof.

An opening 36B of the tube elements 31 to 34 shown in Fig. 6(B) has approximate rectangular-shaped groove extending in radial direction from the center of the tube elements 31 to 34 located by an even disposition of 120 degrees forming an approximate Y-shaped configuration, the groove having an inner side with concave and convex portion.

An opening 36C of the tube elements 31 to 34 shown in Fig. 6(C) has no concave and convex portion on the inner side unlike the opening 36B shown in Fig. 6(B) and the radial length of respective rectangular shape is shortened.

An opening 36D of the tube elements 31 to 34 shown in Fig. 6(D) has three thin triangle and circular projections alternatively projecting from an inner circumference of a circular base hole toward the center thereof.

An opening 36E of the tube elements 31 to 34 shown in Fig. 6(E) has branch-shaped projections with slightly deformed configuration of Fig. 6(A) and internal-gear-shaped concave and convex portion inside the base hole.

The deformation effect of deformed opening of the tube elements 31 to 34 is prominent when the deformation degree represented by square root of inner circumferential dimension of opening/opening cross-sectional area exceeds 7, and the above respective opening 36A to 36E have great deformation degree exceeding 7.

Incidentally, the above-described tube elements 31 to 34 having minute and deformed opening configuration can be molded using a die shown in Japanese Patent Application Laid-Open No. Sho 51-21927. In the molding method, a die for monofilament having a multiple of resin introduction hole provided to an area substantially the same as the outer diameter of the tube elements 31 to 34 and having no hole to a portion corresponding to the opening 36A to 36E is used. A molten resin monofilament is extruded from the introduction holes and the multiple of close monofilament is fused to obtain the tube elements 31 to 34 with minute and deformed configuration. However, the manufacturing method of the tube elements 31 to 34 is not limited to the method.

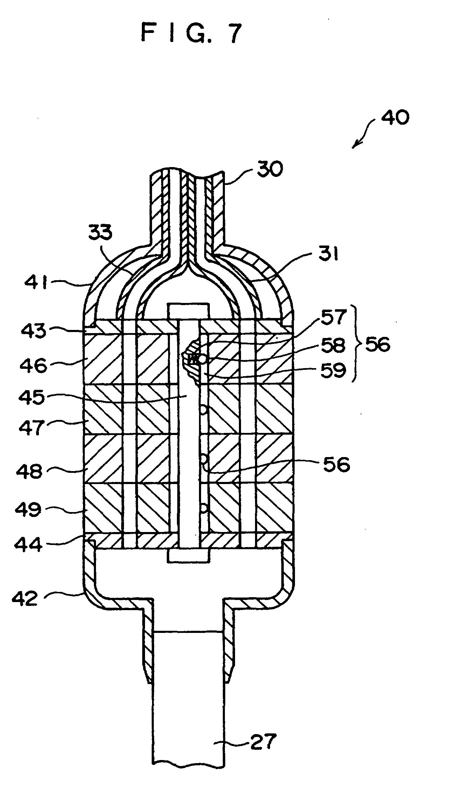

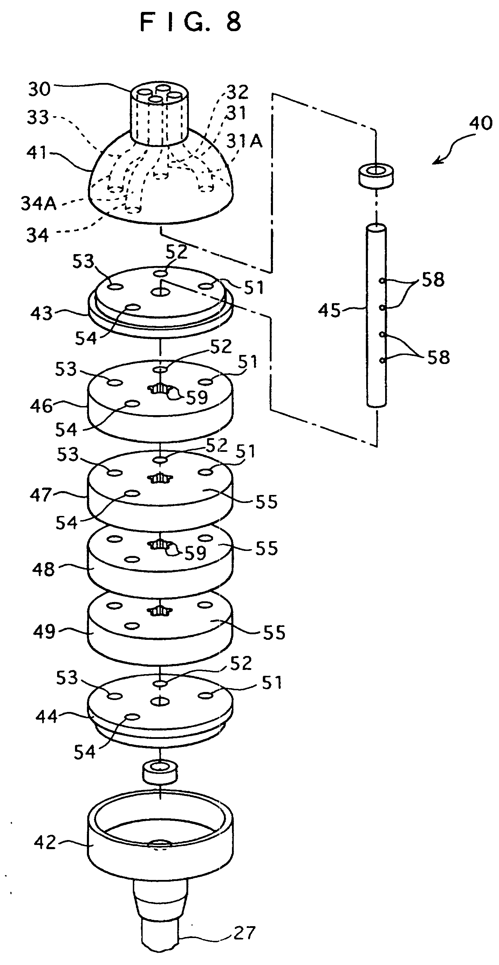

As shown in Fig. 7 and 8, the flow rate switching device 40 has an inflow case 41 having an inflow hole (upper end in the figure) connected to the fluid delivering tube 30, an outflow case 42 having an outflow hole (lower end in the figure) connected to the tube 27, a holding plate 43 and 44 attached to an open end of respective cases 41 and 42, a support bar 45 extending at the center of the holding plate 43 and 44, and four flow path opening plates 46, 47, 48 and 49 rotatable around the support bar 45 mutually abutting between the holding plate 43 and 44. Incidentally, polyethylene (PE), polycarbonate (PC), polypropylene (PP) and the like can be used as a material therefor.

Four fluid passage holes 51, 52, 53 and 54 are respectively formed on the holding plates 43 and 44, and the flow path opening plates 46 to 49 on an identical circumference with the support bar 45 at an interval of 72 degrees. Incidentally, a closing area 55 for closing only one of flow paths 31A to 34A in accordance with angular position is formed between the fluid passage holes 51 and 54 of the flow path opening plates 46 to 49.

Respective tube elements 31 to 34 of the fluid delivering tube 30 are connected to the respective fluid passage holes 51 to 54 of the holding plate 43.

A rotative self-positioning mechanism 56 for positioning the respective flow path opening plates 46 to 49 in accordance with the angle in which the flow passage hole 51 to 54 and the closing area 55 of the respective flow path opening plates 46 to 49 are sequentially accorded with the flow passage holes 51 to 54 of the holding plates 43 and 44, is provided between the support bar 45 and the respective flow path opening plates 46 to 49. The rotative self-positioning mechanism 56 includes a ball 58 biased to project outward by a spring 57 to a position opposing the respective flow path opening plates 46 to 49 of the support bar 45, and an engaging groove 59 formed on the inner circumference of the respective opening plates 46 to 49 for the ball 58 to engage selectively by the interval of 72 degrees.

A using method of the present embodiment will be described below.

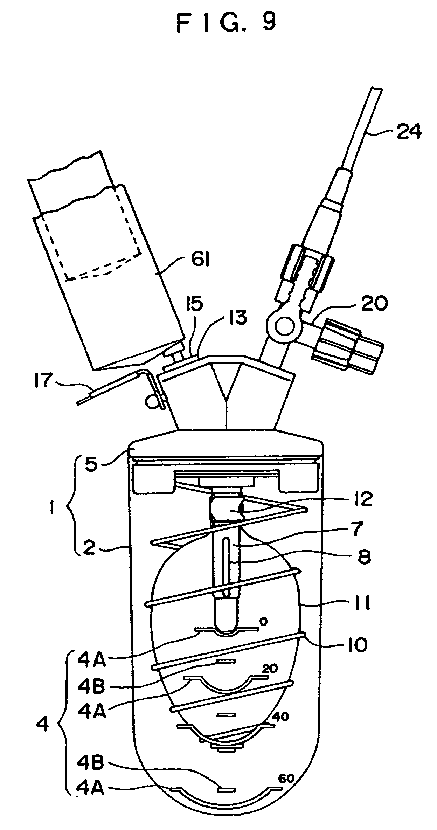

When the medical fluid is received in the rubber elastic film 11, the cap 17 is detached from the valve cylinder 15 of the check valve 13 and a pointed end of a syringe 61 in which the medical fluid is contained is inserted in the valve cylinder 15 of the check valve 13 as shown in Fig. 9. When the medical fluid inside the syringe 61 is pushed out at this state, the medical fluid is received inside the rubber elastic film 11 through the check valve 13 to expand the rubber elastic film 11. The spring 10 is stretched in proportion to the expansion of the rubber elastic film 11, so that the amount of the medical fluid received inside the rubber elastic film 11 can be read by the value of the scale 4 corresponding to the pointed end of the spring 10.

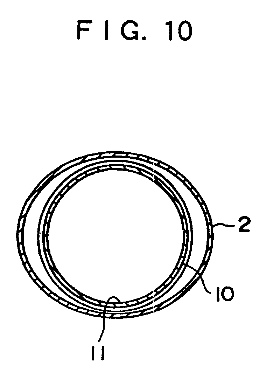

Subsequently, the rubber elastic film 11 abuts an inside of the cylinder body 2 of the protection case 1. Since the cross-section of the protection case 1 is formed in oval configuration as shown in Fig. 10, the contact area of the rubber elastic film 11 with the protection case 1 can be reduced as compared with circular configuration. Further, since the air flow inside the protection case 1 can be ensured, the air inside the cylinder body 2 are discharged to the outside through the water-repellant breathable filter 9A in accordance with the expansion of the rubber elastic film 11. Accordingly, the medical fluid can be accurately delivered little by little, and the attachment position of the air vent 9 is not restricted. After receiving the medical fluid, the valve seat 14 of the check valve 13 is shut when the pointed end of the syringe 61 is pulled out from the check valve 13. Accordingly, the medical fluid inside the rubber elastic film 11 does not leak to the outside.

Next, a syringe needle 29 is attached to the connector 28 at the distal end of the tube 27 and entered to human body. When the cock 23 of the three-direction valve 20 is opened, the medical fluid is sequentially introduced to the human body through the fluid delivering tube 30, the flow rate switching device 40 and the tube 27 at a small flow rate. Incidentally, the small flow rate of the present invention usually refers to around 0.8ml/hr. However, the flow rate can be optionally determined in accordance with configuration of the deformed opening, length and viscosity of the medical fluid and is not restricted to the above flow rate.

When the flow rate is changed, the respective flow path opening plates 46 to 49 or the flow rate switching device 40 are rotated.

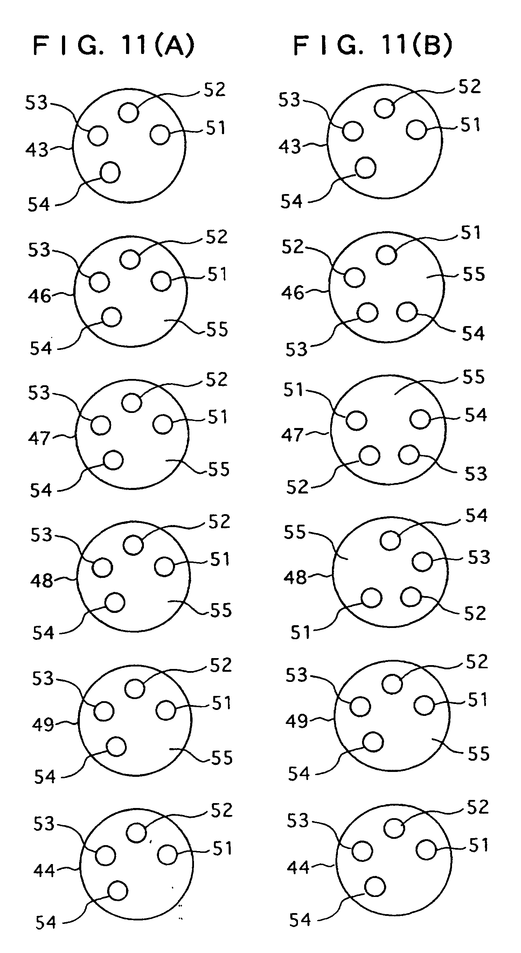

For example, when the flow path opening plates 46 to 49 are rotated to be a condition shown in Fig. 11(A), since all of the fluid passage holes 51 to 54 of the holding plates 43 and 44 and the respective flow path opening plates 46 to 49 are intercommunicated, the flow rate is a sum of the fluid passage rate of the flow paths 31A to 34A of the respective tube elements 31 to 34. For instance, if the fluid passage rates of respective flow path 31A to 34A of the tube elements 31 to 34 are 2.0 ml/hr, 0.8 ml/hr, 0.7 ml/hr and 0.5 ml/hr respectively, the (total) flow rate is 4.0 ml/hr.

When the flow path opening plates 46 to 49 are further rotated as shown in Fig. 11(B), only the fluid passage hole 54 of the holding plates 43 and 44 is intercommunicated through the respective fluid passage hole of the respective flow path opening plates 46 to 49, the flow rate is the fluid passage rate of the flow path 34A of the tube element 34. Therefore, the flow rate is 0.5 ml/hr.

When all the medical fluid in the rubber elastic fluid 11 is injected into the human body changing the flow rate as necessary, the medical fluid is filled in the rubber elastic film 11 similarly to the above description and the above-described operation is repeated. Incidentally, in order to remove air inside the rubber elastic film 11 before entering the syringe needle 29 to the human body, the protection case 1 is set upright with the lid body 5 upward and leave it while the cock 23 is made open.

According to the above-described embodiment, following effects can be obtained.

Since a tube having a predetermined length and a plurality of flow path 31A to 34A mutually parallel along the longitudinal direction thereof is used as the fluid delivering tube 30 and a flow rate switching device 40 for selectively circulating the medical fluid to the flow paths 31A to 34A is provided, the medical fluid can be selectively circulated to the plurality of flow path 31A to 34A by the flow rate switching device 40. Further, since the fluid passage rates of the respective flow paths 31A to 34A mutually differ, the flow rate can be changed by selecting the flow paths 31A to 34A. Accordingly, the flow rate can be changed easily.

Since the plurality of the paths 31A to 34A is provided in a single fluid delivering tube 30, the fluid supplying apparatus is not inconvenient for carrying and storing and usability thereof is not deteriorated.

The flow rate switching device 40 has the inflow case 41 with the fluid delivering tube 30 connected thereto, the outflow case 42, the holding plates 43 and 44 attached to respective open end of the case 41 and 42, and the four flow path opening plates 46 to 49 rotatably provided between the holding plates 43 and 44 being abutted with each other. The holding plates 43 and 44 and the respective opening plates 46 to 49 have a plurality of the fluid passage holes 51 to 54 corresponding to the flow paths 31A to 34A on the common circumference with the support bar 45 as the center and the closing area 55 is provided between the fluid passage holes 51 and 54 of the respective flow path opening plates 46 to 49. Accordingly, the flow path 31A to 34A can be selectively used by selectively closing the plurality of flow paths 31A to 34A with the closing area 55 while rotating the respective flow path opening plates 46 to 49, thereby conducting flow rate change with a simple operation.

The flow rate can be minutely changed in a multi-stage by selecting the number of the selected flow path and/or the combination of the flow paths having different fluid passage rate. Incidentally, when the fluid passage rate of the flow paths 31A to 34A of the tube elements 31 to 34 is set as 2.0 ml/hr, 0.8 ml/hr, 0.7 ml/hr and 0.5 ml/hr respectively, the flow rate can be minutely changed in a multi-stage within the range of the minimum flow rate of 0.5 ml/hr and the maximum flow rate of 4.0 ml/hr.

Since the rotative self-positioning mechanism 56 is provided between the support bar 45 and the respective flow path opening plates 46 to 49, the respective flow path opening plates 46 to 49 can be positioned at a predetermined angle (72 degrees) with a click. Accordingly, the fluid passage holes 51 to 54 of the respective flow path opening plates 46 to 49 and the closing area 55 can be precisely adjusted to the respective flow paths 31A to 34A. Accordingly, the flow rate can be changed accurately.

Since the rotative self-positioning mechanism 56 includes a ball 58 biased to project outward by a spring 57 to a position opposing the respective flow path opening plates 46 to 49 of the support bar 45, and an engaging groove 59 formed on the inner circumference of the respective opening plates 46 to 49 for the ball 58 to engage selectively by the interval of 72 degrees, the rotative self-positioning mechanism 56 can be manufactured in a simple and inexpensive arrangement.

Since the tube 30 having thereinside the elongated thermoplastic-resin-made tube elements 31 to 34 with deformed openings 36A to 36E is used as a flow rate control means instead of conventional short tube having circular opening, the flow rate can be controlled precisely by optionally setting the configuration of the opening and the tube length. When a conventional tube with circular opening is used as a conduit and a dust of a larger size than the inner diameter thereof is contained in the medical fluid or the medical fluid is likely to be coagulated, the medical fluid flow tend to be entirely stopped because the opening is shut. On the other hand, since the tube 30 having predetermined tube elements 31 to 34 having deformed opening configuration is employed, the long side of the deformed opening 36A to 36E is not shut by dust. Accordingly, the blocking of the opening 36A to 36E can be more effectively prevented than the conventional tube having circular opening when the medical fluid contains foreign substance such as dust and solid substance.

Though the tube with the conduit of the conventional tube having circular opening tends to be bent to shut by the weight of a lying patient, the tube elements 31 to 34 having deformed opening according to the present embodiment is tough against bend and is not likely to be shut even when the weight is applied. Therefore, the fluid delivering apparatus without shutting is safer and is significantly effective in a medical field where safety is of importance.

Furthermore, since the conduit function and the flow rate control function are both performed by the tube elements 31 to 34, the structure is more simple than the conventional combination of conduit tube and the flow rate control means.

When the conventional stainless thin tube and glass thin tube is used for performing both the conduit function and the flow rate control function, they are apt to be cracked, broken and difficult to be handled for being too thin. However, since the tube 30 made of thermoplastic resin is used in the present embodiment, deformed opening 36A to 36E having the predetermined configuration is easy to be manufactured, handled easily and both of the conduit function and the minute flow rate control function can be performed.

The number of the flow path formed inside the single fluid delivering tube 30 is not limited to four of the aforesaid embodiment, but more than one flow path is preferably provided inside the tube 30.

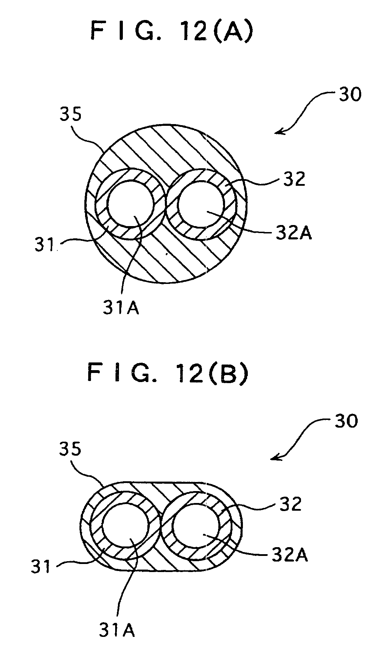

For instance, when two flow paths are provided, two tube elements 31 and 32 having flow paths 31A and 32A thereinside may be bundled and outside of the tube elements may be covered with the covering member 35 in a circular cross-section, as shown in Fig. 12(A). Alternatively, as shown in Fig. 12(B), two tube elements 31 and 32 having flow paths 31A and 32A thereinside may be bundled and outside of the tube elements may be covered with the covering member 35 in an ellipse cross-section. In the above arrangement, the fluid passage rate of the respective flow paths 31A and 32A may be the same or different with each other. Further, since either one of the flow paths 31A and 32A may be opened and closed in the flow path selecting means (flow rate switching device), the structure can be simplified.

In the aforesaid embodiment, the plurality of tube elements 31 to 34 is bundled and the outside thereof is unitedly covered by the covering member 35 to make a single tube. However, a thin core member may be set at a predetermined position in forming the tube and resin may be filled to the outside, so that the tube having thereinside a plurality of flow paths can be integrally formed after removing the core member.

The present invention can be applied to medical fluid injecting apparatus for wide range of medical field such as injecting to veins and urinary organs, and application to obstetrics and gynecology. The present invention can also be used for injecting medical fluid and nutrients to living body such as animals and fishes.

The present invention can also be used for gradually delivering water, (fluid) nutrients and medical fluid (insecticide) to a plant. For instance, in order to gradually supply the water or the (fluid) nutrients in raising vegetables and flowers, it is only required that the distal end of the tube 30 or a needle attached to the distal end of the tube 30 is buried to the grounds around the vegetables and flowers. In the arrangement, the opening of the tube 30 is not shut even when the tube 30 is treaded on to bend the tube 30, thereby not interrupting the delivery of the fluid. When the medical fluid is injected into trees, it is only required that the protection case 1 is hanged to the trees by an appropriate means and the needle at the distal end of the tube 30 is entered to the trees. In this case, the fluid is not limited to flow out downward from the hanged protection case 1 but the medical fluid can be injected to an upper position of the protection case 1.

Further, the present invention can be applied for gradually delivering medical fluids such as antibiotics, (fluid) bait and (fluid) nutrients for water grass to fish aquarium. In this case, the distal end of the tube 30 may be positioned in the aquarium without attaching the needle.

## Claims

1. A fluid supplying apparatus comprising:
a fluid container (11) which contains fluid in use;
a fluid delivering tube (30), arranged to deliver, in use, the fluid therethrough, the fluid delivering tube being formed in a predetermined length and having thereinside a plurality of flow paths mutually extending parallel along a longitudinal direction thereof,
a flow path selecting means (40), arranged to selectively circulate, in use the fluid contained in the fluid container into the plurality of flow paths,
**characterised in that** the flow path selecting means comprises:
a case (41,42) having an inflow hole with the fluid delivering tube connected thereto;
an outflow hole opposite to the inflow hole, and
a plurality of flow path opening plates (46,47,48,49) provided pivotably around a center of identical rotation axis and mutually abutted;
wherein each flow path opening plate has a plurality of flow passage holes (51,52,53,54) formed on a common circle having the rotation axis as a center thereof and a closing area (55) for closing only one of the flow paths in accordance with rotational angle position.

2. The fluid supplying apparatus according to claim 1, wherein each of the plurality of flow paths differs in fluid passage rate.

3. The fluid supplying apparatus according to any of claim 1 or claim 2, wherein the plurality of flow paths is formed of a thermoplastic-resin tube element (31,32,33,34) of a predetermined length having an opening with deformed cross-section.

## Patentansprüche

1. Abgabevorrichtung für Flüssigkeiten, welche enthält:
einen Flüssigkeitsbehälter (11), welcher zu verwendende Flüssigkeit enthält;
eine Flüssigkeitsabgaberöhre (30), die so ausgeführt ist, dass sie im Gebrauch die Flüssigkeit durch sich hindurch abgibt, wobei die Flüssigkeitsabgaberöhre mit einer vorbestimmten Länge ausgebildet ist und in ihrem Inneren eine Vielzahl von Flüssigkeitspfaden aufweist, die sich jeweils wechselseitig parallel längs einer Längsrichtung hiervon erstrecken,
Flusspfadauswahlmittel (40), die so angeordnet sind, dass die im Flüssigkeitsbehälter enthaltene Flüssigkeit bei Gebrauch wahlweise in die Vielzahl von Flüssigkeitspfaden eingespeist wird,
**dadurch gekennzeichnet, dass** die Flusspfadauswahlmittel umfassen:
ein Gehäuse (41, 42) mit einem Einflussloch, mit dem die Flüssigkeitsabgaberöhre verbunden ist;
ein Ausflussloch, gegenüberliegend vom Einflussloch, und
eine Vielzahl von Flusspfadöffnungsplatten (46, 47, 48, 49), die drehbar um ein Zentrum einer identischen Drehachse angeordnet sind, und die wechselweise aneinanderstoßen;
wobei eine jede Flusspfadöffnungsplatte eine Vielzahl von Flussdurchlasslöchern (51, 52, 53, 54) aufweist, die auf einem gemeinsamen Kreis ausgebildet sind, welcher die Drehachse als Mittelpunkt aufweist, sowie eine Verschlussfläche (55) zum Schließen von nur einem der Flusspfade in Übereinstimmung mit der Stellung des Drehwinkels.

2. Abgabevorrichtung für Flüssigkeiten nach Anspruch 1, wobei ein jeder der Vielzahl von Flusspfaden sich in der Flüssigkeitsdurchlassrate unterscheidet.

3. Abgabevorrichtung für Flüssigkeiten nach einem der Ansprüche 1 oder 2, wobei die Vielzahl von Flusspfaden ausgebildet ist aus einem Röhrenelement (31, 32, 33, 34) aus thermoplastischem Kunststoff, mit einer vorbestimmten Länge, welche eine Öffnung mit verformtem Querschnitt aufweist.

## Revendications

1. Appareil de distribution de fluide comprenant :
un récipient pour fluide (11) qui contient un fluide lors de l'utilisation ;
un tube d'acheminement de fluide (30), agencé pour acheminer, lors de l'utilisation, le fluide à travers ce dernier, le tube d'acheminement du fluide étant formé sur une longueur prédéterminée et ayant à l'intérieur une pluralité de chemins d'écoulement s'étendant mutuellement de façon parallèle le long d'une direction longitudinale de celui-ci,
un moyen de sélection de chemin d'écoulement (40) agencé pour faire circuler de manière sélective, lors de l'utilisation, le fluide contenu dans le récipient pour fluide dans la pluralité de chemins d'écoulement,
**caractérisé en ce que** le moyen de sélection de chemin d'écoulement comprend :
un boîtier (41, 42) ayant un trou d'arrivée, le tube d'acheminement du fluide étant raccordé à ce dernier ;
un trou de sortie opposé au trou d'arrivée, et
une pluralité de plaques d'ouverture de chemins d'écoulement (46, 47, 48, 49) situées de manière pivotante autour d'un centre d'axe de rotation identique et mutuellement contiguës ;
dans lequel chaque plaque d'ouverture de chemins d'écoulement comporte une pluralité de trous de passage (51, 52, 53, 54) formés sur un cercle commun ayant l'axe de rotation comme centre et une zone de fermeture (55) destinée à fermer uniquement l'un des chemins d'écoulement conformément à la position angulaire de rotation.

2. Appareil de distribution de fluide selon la revendication 1, dans lequel chaque chemin d'écoulement de la pluralité de chemins d'écoulement diffère par la vitesse de passage du fluide.

3. Appareil de distribution de fluide selon l'une quelconque des revendications 1 et 2, dans lequel la pluralité de chemins d'écoulement sont formés d'un élément de tube en résine thermoplastique (31, 32, 33, 34) d'une longueur prédéterminée ayant une ouverture à section transversale déformée.
